# EUROPÄISCHE PATENTSCHRIFT

(11) **EP 2 486 400 B1**
(45) Veröffentlichungstag und Bekanntmachung des Hinweises auf die Patenterteilung: **15.05.2013**
(21) Anmeldenummer: 10740551.6
(22) Anmeldetag: 31.07.2010
(51) Int. Cl.: G01N 33/28, G01N 15/14

(54) **VORRICHTUNG ZUR PARTIKELMESSUNG IN HYDRAULIK-, GETRIEBE- UND SCHMIERÖLEN**
DEVICE FOR MEASURING PARTICLES IN HYDRAULIC, MECHANICAL, AND LUBRICATING OILS
DISPOSITIF DE MESURE DE PARTICULES DANS LES HUILES HYDRAULIQUES, DANS LES HUILES D'ENGRENAGE, ET DANS LES HUILES DE GRAISSAGE

(30) Priorität: 05.10.2009 DE 102009048271
(43) Veröffentlichungstag der Anmeldung: 15.08.2012
(73) Patentinhaber: Robert Bosch GmbH, 70469 Stuttgart (DE)
(72) Erfinder: TRAHAN, Christian, 68782 Brühl (DE); HORNUNG, Raphael, 66440 Blieskastel (DE); KÖHLER, Tobias, 69214 Eppelheim (DE)
(74) Vertreter: Thürer, Andreas
(86) Internationale Anmeldenummer: PCT/EP2010/004700
(87) Internationale Veröffentlichungsnummer: WO 2011/042081

(56) Entgegenhaltungen:
- US-A- 6 151 108
- US-B1- 6 555 360
- US-B1- 6 582 661

## Beschreibung

Die vorliegende Erfindung betrifft eine Vorrichtung zur Partikelmessung in Hydraulik-, Getriebe-, Schmierölen und dergleichen Fluiden/Betriebsmedien gemäß dem Oberbegriff des Patentanspruchs 1, dessen Merkmale aus der Druckschrift US 6,555,360 B1 bekannt sind.

Um ein repräsentatives Bild der Feststoffverschmutzung von Betriebsmedien beispielsweise in Hydraulik- oder Schmierungsanlagen zu erhalten, muss man oft an Anlagenmassorten Partikelzählungen durchführen, die ein sehr mit Gasblasen beladenes Betriebsmedium anbieten. Beispiele für solche Messorte sind Messstellen an Rücklaufleitungen und an Getriebeschmieranlagen, insbesondere bei Windkraftanlagen.

Herkömmliche optische Partikelzählverfahren können Feststoffpartikel nicht von Gasblasen unterscheiden. Alternative Partikelzählverfahren, die diese Querempfindlichkeit nicht besitzen, haben jedoch folgende Nachteile:
- Ein bekanntes Siebverfahren ist nicht auf einfache Weise automatisierbar.
- Ein bekanntes induktives Verfahren reagiert nur zuverlässig auf metallische Partikel mit einer vorbestimmten Partikelgröße.

Um Feststoffverschmutzungen unterhalb einer Partikelgröße von 100µm bis hinab auf 4 µm Äquivalentdurchmesser zu detektieren, wird der Einsatz von optischen Partikelzählern unumgänglich, wobei parallel hierzu jedoch Maßnahmen ergriffen werden müssen, um den Einfluss von Gasblasen in den Hintergrund zu drängen.

Ein Abtrennen von emulgiertem Gas vom Betriebsmedium ist gerätetechnisch sehr aufwändig und deshalb unwirtschaftlich. Als einfache Lösung bietet es sich deshalb an, durch Druckerhöhung das mitgeführte Gas im Medium zu lösen (bei einer Druckerhöhung von 1 bar auf 100 bar lassen sich in einem Liter Öl etwa neun Liter Luft lösen). Außerdem werden die Gasblasen komprimiert, so dass diese in ihrer Größe teilweise unter die Nachweisgrenze für die Partikel gelangen.

Eine bekannte Vorrichtung, in der ein optischer Partikelzähler Verwendung findet, hat in der Regel einen Sensor, der auf die Anwesenheit von Partikeln in einer von einem Fluidstrom eines beispielsweise viskosen Mediums durchströmten Messzone optisch anspricht und ein entsprechendes Erfassungssignal an eine elektronische Auswerteeinrichtung abgibt. Aus dem Stand der Technik, beispielsweise gemäß der DE 103 43 457 B3, ist eine Vorrichtung dieser Gattung bekannt. Diese Vorrichtung hat einen Partikelzähler, bei dem mittels eines Sensors ein auswertbares Sensorsignal erzeugt wird. Der Sensor spricht dabei auf die Anwesenheit von Partikeln in einer von einem Fluidstrom eines viskosen Mediums durchströmten Messzone optisch an, wobei vor Eintritt des Fluidstroms in die Sensor-Messzone des Partikelzählers der Eingangsdruck für das viskose Medium mittels einer Einstelleinrichtung angepasst wird.

Als besonders problematisch haben sich dabei jedoch bestimmte Aufschäumvorgänge im Medium erwiesen, die beim Betreiben der jeweiligen Anlage insbesondere beim Zurückführen des Mediums in einen Fluidtank auftreten können, Derartige Gasblasen würden, wie bereits eingangs ausgeführt wurde, zu einer Verfälschung der Partikelmessung führen und sind daher in jedem Fall zu vermeiden bzw. zu eliminieren,

Aus diesem Grund ist die aus dem genannten Stand der Technik bekannte Einstelleinrichtung so eingerichtet, dass im Medium enthaltene Luftblasen auf dem Weg zwischen Fluidtank und Sensor-Messzone infolge eines Druckaufbaus in Lösung gehen, so dass der Partikelzähler nicht im Sinne einer Fehlmessung dahingehende unschädliche Luftblasen als vermeintliche Verschmutzungspartikel erkennt. Um diesen Effekt des Gaslösens im Medium weiter zu verbessern, sieht der bekannte Stand der Technik vor, zwischen einer Hydropumpe, welche das Medium aus dem Fluidtank fördert und druckbeaufschlagt, sowie dem Partikelzähler eine Art Beruhigungsstrecke einzusetzen, wodurch der Strömungsweg zwischen Pumpe und Partikelzähler verlängert und damit der Betrag an in Lösung gehendem Gas erhöht wird.

Trotz dieser Maßnahmen im Stand der Technik ist der negative Einfluss von nicht gelösten Gasblasen im Medium nicht gänzlich eliminiert. Insbesondere berücksichtigt dieser bekannte Stand der Technik nicht vollumfänglich, dass einerseits das Lösen von Gas im Medium eine gewisse Zeit dauert und dass andererseits ein auch nur kurzzeitig auftretender Druckeinbruch zum vollständigen Ausgasen der bereits gelösten Luft führt.

Es ist daher die Aufgabe der vorliegenden Erfindung, eine Vorrichtung zur Partikelmessung bereit zu stellen, die ein verbessertes Messergebnis liefert.

Diese Aufgabe wird durch eine gattungsgemäße Vorrichtung mit den Merkmalen des Patentanspruchs 1 gelöst. Vorteilhafte Ausgestaltungen der Erfindung sind Gegenstand der Unteransprüche.

Durch den Einsatz einer vorzugsweise volumetrisch arbeitenden, gasabscheidenden Förderpumpe, die mit einer separaten Rücklauf- bzw. Leckageleitung ausgestattet/verbunden ist, werden größere Gasblasen bereits aus dem Fluidförderstrom ausgeschieden und gelangen so nicht mehr in die Zuführleitung zum vorzugsweise optischen Partikelzähler,

Die Leitungslänge zwischen Pumpe und Partikelzähler hängt dabei vorzugsweise vom Volumenstrom der Förderpumpe, von der Nennweite der Leitung und der Viskosität des Mediums ab und garantiert die erforderliche Verweilzeit des Mediums vor dem Partikelzähler, um das Gas im Medium zu lösen und derart vor dem Partikelzähler zu verbergen.

Der weiter vorzugsweise angeordnete Druckspeicher sorgt dafür, dass der Druck des Gas- und Feststoff beladenen Mediums (Fluids) nur wenig einbricht, wenn die Förderpumpe keinen konstanten Volumenstrom beim Fördern des Medium-Gas-Gemischs erzeugt. Ein plötzliches Ausgasen und die damit verbundene Verfälschung der Partikelzählung werden auf diese Weise vermieden. Außerdem wird durch die dem Partikelzähler nachgeschaltete Anordnung des Druckspeichers eventuell bereits ausgegastes Gas (Luft) in Richtung zur Pumpe verschoben (zurückgedrängt) und gelangt somit nicht unmittelbar zum Partikelzählsensor.

Das ebenfalls vorzugsweise angeordnete Druckhalteventil bestimmt dabei den Druck in diesem Kreis und kann auf einen festen (vorbestimmten) Wert eingestellt sein.

Die Erfindung wird nachstehend anhand eines bevorzugten Ausführungsbeispiels unter Bezugnahme auf die begleitende Zeichnung näher erläutert. Die einzige Figur zeigt dabei das Schaltbild eines Fluidkreises einer Partikelmessvorrichtung gemäß einem bevorzugten Ausführungsbeispiel der Erfindung.

Die erfindungsgemäße Vorrichtung hat demzufolge eine Förder- oder Fluidpumpe 1, die von einem vorzugsweise elektrischen Motor M angetrieben wird und die über eine Saugleitung 1a ein Medium, beispielsweise Hydrauliköl, Getriebe-/Motorenöl, Schmiermittel oder dergleichen Fluide aus einem Fluidtank T ansaugt. Die Pumpe 1 ist über eine Fluidzuführfeitung 2 mit einem Partikelzähler 3 fluidverbunden, um das angesaugte Medium unter einem bestimmten Druck dem Partikelzähler 3 zuzuführen. Vom Partikelzähler 3 führt eine Rückführleitung 7 zum Fluidtank T zurück, an die unmittelbar stromab zum Partikelzähler 3 ein Druckbehälter oder -speicher 4 über eine Zweigleitung 8 angeschlossen ist. Ferner ist unmittelbar stromab zum Druckspeicher 4 ein Druckhalteventil 5 in die Rückführleitung 7 zwischengeschaltet. Dieses Druckhalteventil 5 ist ein Druckbegrenzungsventil mit einer Feder-belasteten Steuerseite und einer Fluid-beaufschlagten Steuerseite, die über eine Steuerleitung 9 mit der Rückführleitung 7 unmittelbar stromauf zum Druckhalteventil 5 verbunden ist.

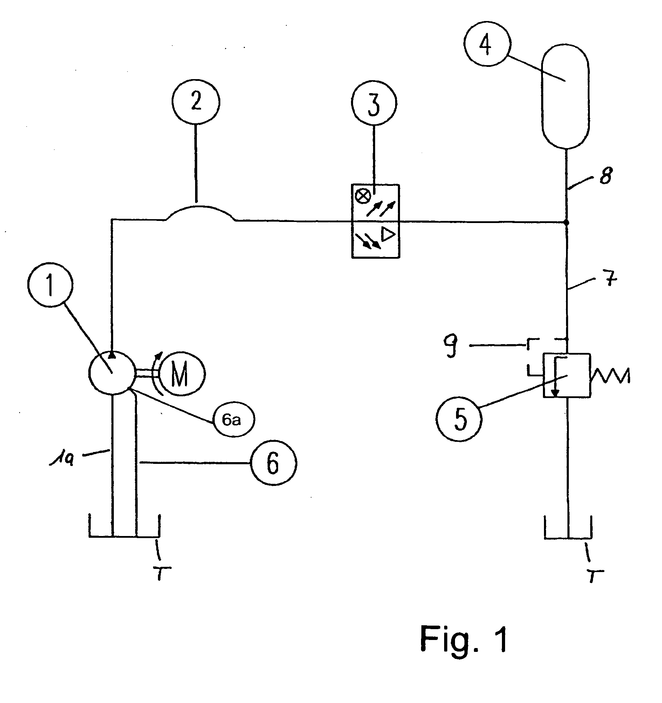

Gemäß der vorliegenden Erfindung zweigt von der Fluidpumpe 1 eine Leckageleitung 6 ab, die an einen Leckölanschluss 6a der Fluidpumpe 1 angeschlossen ist und in den Fluidtank T führt. Alternativ hierzu kann die Pumpe auch intern mit einer derartigen Leckageleitung ausgestattet sein. Bei der Fluidpumpe 1 handelt es sich im Übrigen vorzugsweise um eine Zahnradpumpe, die im Bereich ihrer Antriebswelle die Leckagefluidentlastung aufweist. Eine solche Pumpe ist z.B. in der DE 101 12 660 A1 dargestellt. Gemäß Figur 2 dieses Dokumentes zweigt zwischen einem ersten Dichtungselement und einem zweiten Dichtungselement der Wellendichtung der Antriebswelle ein Leckölkanal ab, der an einem stirnseitigen Leckölanschluss, z.B. in der Nähe der Antriebswelle herausgeführt ist.

Gemäß diesem Ausführungsbeispiel der Erfindung ist die Zahnradpumpe mit ihrer Antriebswelle senkrecht oder schräg montiert, so dass die Antriebswelle nach oben ragt und vorzugsweise aus dem Pumpengehäuse heraussteht. Auch der Leckölanschluss 6a ist damit nach oben orientiert.

Die Wirkungsweise der erfindungsgemäßen Vorrichtung lässt sich wie Folgt umschreiben:
Bei Ansaugen von Medium aus dem Fluidtank T werden auch Gasblasen mit angesaugt, die sich durch Aufschäumvorgänge im Medium gebildet und im Fluidtank T angereichert haben. Zudem tritt auch durch den Unterdruck beim Ansaugen weiteres Gas aus dem Medium aus und sammelt sich in Blasen im Eingangskanal der Pumpe 1. Beim Fördern des Mediums durch die Pumpe 1 können bereits große Anteile der Gasblasen über den Pumpen internen Leckageanschluss entweichen.
Die Gasblasen wandern während des Komprimiervorgangs in der Pumpe 1 entlang der schräg- oder senkrecht gestellten Antriebswelle nach oben, entweichen über den Leckölanschluss 6a, und werden so dem Medium entzogen.

Wie bereits im Stand der Technik vorgesehen ist, wird auch bei der vorliegenden Erfindung ein weiterer Anteil der Gasblasen durch das Komprimieren des Mediums in diesem gelöst und stört so die Partikelzählung nicht mehr,

Die Länge der Fluidzuführleitung 2 ist in Abhängigkeit des verwendeten Mediums (dessen Viskosität) sowie ggf. der Fördermenge der Pumpe und des Durchmessers der Fluidzuführleitung 2 so bestimmt, dass ein Beruhigungseffekt auf das unter Druck gesetzte Medium ausgeübt wird und die entsprechend notwendige Zeit zum Lösen der Luft durch die Länge der Fließstrecke bereit gestellt wird. So wird z.B. bei einer Leitung von 4 Millimeter Durchmesser eine Leitungslänge zwischen der Förderpumpe 1 und dem Partikelzähler 3 von mindestens einem Meter, vorzugsweise von 2 Metern angestrebt,

Stromab zu der Messstelle des Partikelzählers 3 ist der Druckspeicher 4 vorgesehen, der den Druck in der Messstelle stabilisiert. Hierdurch werden ggf. während eines Druckeinbruchs (aufgrund von Ansaugen von Gasblasen) entstandene Ausgasungen in Richtung zur Pumpe 1 verschoben und können so das Messergebnis nicht verfälschen. Des Weiteren können Schwankungen in den Messergebnissen verringert werden, die durch Druckpulsation, verursacht durch die Fluidpumpe 1, entstehen.

### Bezugszeichenliste

| | |
|---|---|
| Förderpumpe | 1 |
| Ansaugleitung | 1a |
| Fluidzuführleitung | 2 |
| Partikelzähler | 3 |
| Druckspeicher | 4 |
| Druckhalteventil | 5 |
| Leckageleitung | 6 |
| Leckölanschluss | 6a |
| Rückführleitung | 7 |
| Zweigleitung | 8 |
| Steuerleitung | 9 |

## Patentansprüche

1. Vorrichtung zur Partikelmessung in stark mit Gas, insbesondere Luft beladenem Medium, umfassend eine Förderpumpe (1), eine Zuführleitung (2) und einen Partikelzähler (3), die so angeordnet sind, dass dem Partikelzähler (3) das vorzugsweise viskose Medium durch die Förderpumpe (1) über die Zuführleitung (2) zugeführt wird, **dadurch gekennzeichnet, dass** die Förderpumpe (1) eine Öffnung (6a) in Form eines separat zur Zuführleitung (2) ausgebildeten Leckölanschlusses besitzt, über welchen aufgrund seiner Anordnung und Orientierung im Betrieb der Förderpumpe (1) Gas aus dem durch die Förderpumpe (1) geförderten Medium abführbar ist.

2. Vorrichtung nach Anspruch 1, **dadurch gekennzeichnet, dass** die Förderpumpe (1) eine volumetrisch arbeitende Fluidpumpe, insbesondere eine Zahnradpumpe ist.

3. Vorrichtung nach Anspruch 1 oder 2, **dadurch gekennzeichnet, dass** der Partikelzähler (3) einen optischen Sensor hat.

4. Vorrichtung nach einem der vorstehenden Ansprüche, **dadurch gekennzeichnet, dass** die Leitungslänge zwischen der Förderpumpe (1) und dem Partikelzähler (3) in Abhängigkeit vom Volumenstrom der Förderpumpe (1), von der Nennweite der Zuführleitung (2) und der Mediumsviskosität bestimmt ist.

5. Vorrichtung nach einem der vorstehenden Ansprüche, **dadurch gekennzeichnet, dass** die Leitungslänge zwischen der Förderpumpe (1) und dem Partikelzähler (3) die notwendige Zeit zum Auflösen der Luft im Fluid bereit stellt.

6. Vorrichtung nach einem der vorstehenden Ansprüche, **dadurch gekennzeichnet, dass** dem Partikelzähler (3) ein Druckspeicher (4) unmittelbar nachgeschaltet ist.

7. Vorrichtung nach einem der vorstehenden Ansprüche, **gekennzeichnet durch** ein Druckhalteventil (5), das in einer vom Partikelzähler (3) abgehenden Rückführleitung angeordnet und vorzugsweise auf einen festen Wert voreingestellt ist.

8. Vorrichtung nach einem der vorstehenden Ansprüche, **dadurch gekennzeichnet, dass** die Öffnung (6a) in der Nähe einer Antriebswelle der Förderpumpe ausgebildet ist.

9. Vorrichtung nach Anspruch 8, **dadurch gekennzeichnet, dass** die Pumpe mit der Antriebswelle und damit der Öffnung (6a) nach oben montiert ist.

10. Vorrichtung nach Anspruch 4 oder 5, **dadurch gekennzeichnet, dass** die Leitungslänge mindestens 1 Meter, vorzugsweise 2 Meter beträgt.

11. Vorrichtung nach Anspruch 4, 5, oder 10 **dadurch gekennzeichnet, dass** der Durchmesser des Leitungsabschnitts zwischen der Förderpumpe (1) und dem Partikelzähler (3) etwa 4 Millimeter beträgt.

12. Vorrichtung nach Anspruch 1, **dadurch gekennzeichnet, dass** die Förderpumpe (1) für eine schräge oder vertikale Montage angepasst ist.

## Claims

1. Device for measuring particles in a medium heavily laden with gas, in particular air, comprising a feed pump (1), a supply line (2) and a particle counter (3) which are arranged such that the preferably viscous medium is supplied to the particle counter (3) by the feed pump (1) via the supply line (2), **characterized in that** the feed pump (1) possesses an orifice (6a) in the form of a leakage-oil connection which is formed separately from the supply line (2) and via which, by virtue of its arrangement and orientation, gas can be discharged, when the feed pump (1) is in operation, out of the medium conveyed by the feed pump (1).

2. Device according to Claim 1, **characterized in that** the feed pump (1) is a volumetrically operating fluid pump, in particular a gear pump.

3. Device according to Claim 1 or 2, **characterized in that** the particle counter (3) has an optical sensor.

4. Device according to one of the preceding claims, **characterized in that** the line length between the feed pump (1) and the particle counter (3) is determined as a function of the volumetric flow of the feed pump (1), of the nominal width of the supply line (2) and of the medium viscosity.

5. Device according to one of the preceding claims, **characterized in that** the line length between the feed pump (1) and the particle counter (3) affords the time necessary for dissolving the air in the fluid.

6. Device according to one of the preceding claims, **characterized in that** the particle counter (3) is followed directly by a pressure accumulator (4).

7. Device according to one of the preceding claims, **characterized by** a pressure-holding valve (5) which is arranged in a return line emanating from the particle counter (3) and which is preferably preset to a fixed value.

8. Device according to one of the preceding claims, **characterized in that** the orifice (6a) is formed in the vicinity of a driveshaft of the feed pump.

9. Device according to Claim 8, **characterized in that** the pump is mounted with the driveshaft and consequently the orifice (6a) upwards.

10. Device according to Claim 4 or 5, **characterized in that** the line length amounts to at least 1 metre, preferably 2 metres.

11. Device according to Claim 4, 5 or 10, **characterized in that** the diameter of the line section between the feed pump (1) and the particle counter (3) amounts to about 4 millimetres.

12. Device according to Claim 1, **characterized in that** the feed pump (1) is adapted for oblique or vertical mounting.

## Revendications

1. Dispositif de mesure de particules dans un milieu fortement chargé de gaz, en particulier d'air, comprenant une pompe de refoulement (1), une conduite d'alimentation (2) et un compteur de particules (3), qui sont disposés de telle sorte que le milieu de préférence visqueux soit acheminé au compteur de particules (3) par la pompe de refoulement (1) par le biais de la conduite d'alimentation (2), **caractérisé en ce que** la pompe de refoulement (1) possède une ouverture (6a) sous forme de raccord d'huile de fuite réalisé séparément de la conduite d'alimentation (2), par le biais duquel raccord d'huile de fuite, du fait de son agencement et de son orientation pendant le fonctionnement de la pompe de refoulement (1), du gaz peut être évacué hors du milieu refoulé par la pompe de refoulement (1).

2. Dispositif selon la revendication 1, **caractérisé en ce que** la pompe de refoulement (1) est une pompe fluidique volumétrique, notamment une pompe à engrenages.

3. Dispositif selon la revendication 1 ou 2, **caractérisé en ce que** le compteur de particules (3) a un capteur optique.

4. Dispositif selon l'une quelconque des revendications précédentes, **caractérisé en ce que** la longueur de la conduite entre la pompe de refoulement (1) et le compteur de particules. (3) est déterminée en fonction du débit volumique de la pompe de refoulement (1), de la largeur nominale de la conduite d'alimentation (2) et de la viscosité du milieu.

5. Dispositif selon l'une quelconque des revendications précédentes, **caractérisé en ce que** la longueur de conduite entre la pompe de refoulement (1) et le compteur de particules (3) fournit le temps nécessaire pour la libération de l'air dans le fluide.

6. Dispositif selon l'une quelconque des revendications précédentes, **caractérisé en ce qu'**un accumulateur de pression (4) est monté directement en aval du compteur de particules (3).

7. Dispositif selon l'une quelconque des revendications précédentes, **caractérisé par** une soupape de maintien de pression (5) qui est disposée dans une conduite de recirculation partant du compteur de particules (3) et qui est de préférence préajustée à une valeur fixée.

8. Dispositif selon l'une quelconque des revendications précédentes, **caractérisé en ce que** l'ouverture (6a) est réalisée à proximité d'un arbre d'entraînement de la pompe de refoulement.

9. Dispositif selon la revendication 8, **caractérisé en ce que** la pompe est montée avec l'arbre d'entraînement et par conséquent avec l'ouverture (6a) vers le haut.

10. Dispositif selon la revendication 4 ou 5, **caractérisé en ce que** la longueur de la conduite est d'au moins 1 mètre, de préférence de 2 mètres.

11. Dispositif selon la revendication 4, 5 ou 10, **caractérisé en ce que** le diamètre de la section de conduite entre la pompe de refoulement (1) et le compteur de particules (3) est d'environ 4 millimètres.

12. Dispositif selon la revendication 1, **caractérisé en ce que** la pompe de refoulement (1) est adaptée pour un montage oblique ou vertical.
